**Europäisches Patentamt**

**European Patent Office**

**Office européen des brevets**

⑩ (11) Publication number: **0 216 573**
**B1**

(12) **EUROPEAN PATENT SPECIFICATION**

(45) Date of publication of patent specification: **22.11.90**

(21) Application number: **86306999.3**

(22) Date of filing: **11.09.86**

(51) Int. Cl.⁵: **C 12 N 15/00,** C 07 H 21/04,
C 12 P 21/00, A 61 K 39/29

(54) **Yeast promoter and method of producing heterologous proteins.**

(30) Priority: **18.09.85 JP 205823/85**
**03.04.86 JP 77422/86**

(43) Date of publication of application:
**01.04.87 Bulletin 87/14**

(45) Publication of the grant of the patent:
**22.11.90 Bulletin 90/47**

(84) Designated Contracting States:
**BE CH DE FR GB LI NL SE**

(56) References cited:
**EP-A-0 100 561**
**EP-A-0 143 081**

(73) Proprietor: **THE GREEN CROSS CORPORATION**
**3-3, Imabashi 1-chome Chuo-ku**
**Osaka-shi (JP)**

(72) Inventor: **Horii, Hajime**
**2-18-3, Segawa**
**Minoo-shi Osaka (JP)**
Inventor: **Mukai, Hiromichi**
**2-5-2-103, Fujisakahigashimachi**
**Hirakata-shi Osaka (JP)**
Inventor: **Tsujikawa, Muneo**
**26-302, Kitafunabashi-cho**
**Hirakata-shi Osaka (JP)**
Inventor: **Kawabe, Haruhide**
**3-29-14, Yamadanishi**
**Suita-shi Osaka (JP)**
Inventor: **Arimura, Hirofumi**
**2-18-1-401, Uenosaka**
**Toyanaka-shi Osaka (JP)**
Inventor: **Suyama, Tadakazu**
**4-3-7, Matsuigaoka Tanabe-cho**
**Tsuzuki-gun Kyoto (JP)**

(74) Representative: **Laredo, Jack Joseph et al**
**Elkington and Fife Beacon House 113 Kingsway**
**London, WC2B 6PP (GB)**

The file contains technical information
submitted after the application was filed and
not included in this specification

# EP 0 216 573 B1

**Description**

BACKGROUND OF THE INVENTION

This invention relates to an improved yeast promoter.

Recently, researches in the field of genetic engineering are flourishing and genetic engineering is of wide application, for example in the production of useful medicines.

At present, strains of *Escherichia coli, Bacillus subtilis* and yeasts, among others, are mainly used as hosts in genetic engineering. Successful expression of heterologous genes for HBsAg, IFN-α and TPA, for instance, has already been realized in yeasts.

It is known that the mechanism of yeast promoter control generally involves control by positive regulatory elements except for the cases of glucose repression and the like, and the presence of a *cis*-acting upstream activation site (UAS) located upstream from a TATA box (serving to fix the transcription initiation site and enhance the transcription efficiency) and serving as a promoter-side regulatory element participating in the above regulation has been suggested (L. Guarente, Cell, *36*, 799, 1984). It has also been shown that when the UAS is replaced with another UAS, the relevant promoter is released from the control of the regulatory system under which it has originally been controlled but comes under the control of a regulatory system associated with the new substitute UAS (L. Guarente et al., Proc. Natl. Acad. Sci. USA, *79*, 7410, 1982; L. Guarente et al., Cell, *36*, 503, 1984; K. Struhl, Proc. Natl. Acad. Sci. USA, *81*, 7865, 1984). Therefore, as a way of improving yeast promoters, there may be considered modifying said systems by UAS substitution or by decontrol from the regulatory systems causing lack of DNA, as well as miniaturization of the promoters.

EP—A1—0 100 561 describes DNA fragments comprising yeast acid phosphatase promoters, specifically *PHO3* and *PHO5* promoter and describes and illustrates inter alia the nuclear tied sequence of the BamHI-Sall restriction fragment of *PHO5* including the *PHO5* promoter region.

EP—A2—0 143 081 describes yeast hydrid vectors comprising a yeast promoter and a tissue plasminogen activator coding region controlled by the promoter. *PHO3* and *PHO5* promoter regions are described and illustrated including inter alia the said nuclear tied sequence of the BamHI-Sall restriction fragment of PHO5. Processes for the production of TPA are also described.

The *PHO5* (yeast repressible acid phosphatase) promoter is one of the yeast promoters. This promoter is used in HBsAg-producing plasmids.

The transcription of the repressible acid phosphatase structural gene to mRNA is repressed by the presence of an inorganic phosphate in the medium and promoted with increasing inorganic phosphate deficiency. For that reason, the use of the *PHO5* promoter in causing expression of heterologous genes is greatly restricted because of the necessity of using a phosphate-free medium or a host yeast in which *PHO5* can function constitutively as a result of mutation in the *PHO5*-regulating system.

For the purpose of eliminating these restrictions, the present inventors deleted an upstream portion of the *PHO5* promoter. In the course of their study, the inventors found that even when the region upstream from the restriction enzyme *BstEII* site located about 70 bp (base pairs) upstream from the TATA box of the *PHO5* promoter is missing, a potent promoter activity is still maintained in inorganic phosphate-containing media. This finding has now led to completion of the present invention.

SUMMARY OF THE INVENTION

The invention thus provides a DNA sequence consisting essentially of that region of the yeast repressible acid phosphatase (*PHO5*) promoter which covers the 173 base pairs (pb) just upstream from the initiation codon for the *PHO5* protein (without counting the initiation codon), and a method of producing heterologous proteins by utilizing such sequence as the promoter.

BRIEF DESCRIPTION OF THE DRAWINGS

In the accompanying drawings,

Fig. 1 shows the construction scheme for a plasmid pGL2061 and

Fig. 2 shows the construction scheme for a plasmid containing a miniaturized *PHO5* promoter.

In the figures, p stands for promoter, t for terminator, Apc^r for ampicillin resistance gene, IR for inverted repeats, — for pJDB207, and HBsAg for gene coding for hepatitis B surface antigen.

DETAILED DESCRIPTION OF THE INVENTION

The construction scheme for the improved *PHO5* promoter according to the invention is as follows:

The *PHO5* promoter to be used in the practice of the invention is one of the known promoters and its base sequence has been disclosed by Bajwa et al. (Nucleic Acids Res., *12*, 7721, 1984). Therefore, any of those plasmids which carry the *PHO5* promoter may serve as the DNA sequence to be used as the starting material for miniaturization. Thus, for instance, the plasmid pGL2061 to be mentioned later herein is suited for use for such purpose.

The *PHO5* promoter gene is subjected to deletion treatment, which comprises cleavage with a specific restriction enzyme, either after isolation of said gene or in the form of a plasmid carrying said gene. The deletion is carried out so that an upstream portion of the *PHO5* promoter is deleted. It is particularly preferable to cleave the *PHO5* promoter at a restriction enzyme site occurring more than about 70 bp

2

upstream from the TATA box of said promoter. A preferred example of the restriction enzyme is *BstE*II. When cleavage is effected with *BstE*II, a 173-bp portion just upstream from the initiation codon for protein remains.

The DNA sequence of the miniaturized (modified) promoter as determined by the Maxam-Gilbert method is as shown in Table 1.

## Table 1

```
-173                              -150
  .     .                 .          .

GTCACCTTACTTGGCAAGGCATATACCCATTTGG

                                    -100
        .           .           .     .

GATAAGGGTAAACATCTTTGAATTGTCGAAATGAAACGTA

        .           .           .           .

TATAAGCGCTGATGTTTTGCTAAGTCGAGGTTAGTATGGC

  -50           .           .           .
   .

TTCATCTCTCATGAGAATAAGAACAACAACAAATAGAGCA

                  -1
        .          .

AGCAAATTCGAGATTACCA
```

This base sequence is about one third in size as compared with the known promoter although it is identical with that of the corresponding known portion.

This miniaturized (modified) *PHO*5 promoter is inserted for repair into a plasmid by treatment with known restriction enzymes and ligase. When the known plasmid pGL2061 is used as the starting material, this is digested with the restriction enzyme *Sal*I, for instance, followed by repair at both ends and ligation in the presence of ligase. In this way, there is obtained a plasmid carrying the HBsAg gene as the structural gene and the miniaturized (modified) *PHO*5 promoter according to the invention.

By inserting a gene coding for a physiologically active, useful substance (protein) into such plasmid at a site downstream from the thus-obtained miniaturized *PHO*5 promoter, transforming a yeast strain with the resulting recombinant plasmid and further causing expression of said gene, the desired protein can be produced efficiently.

### Reference Example

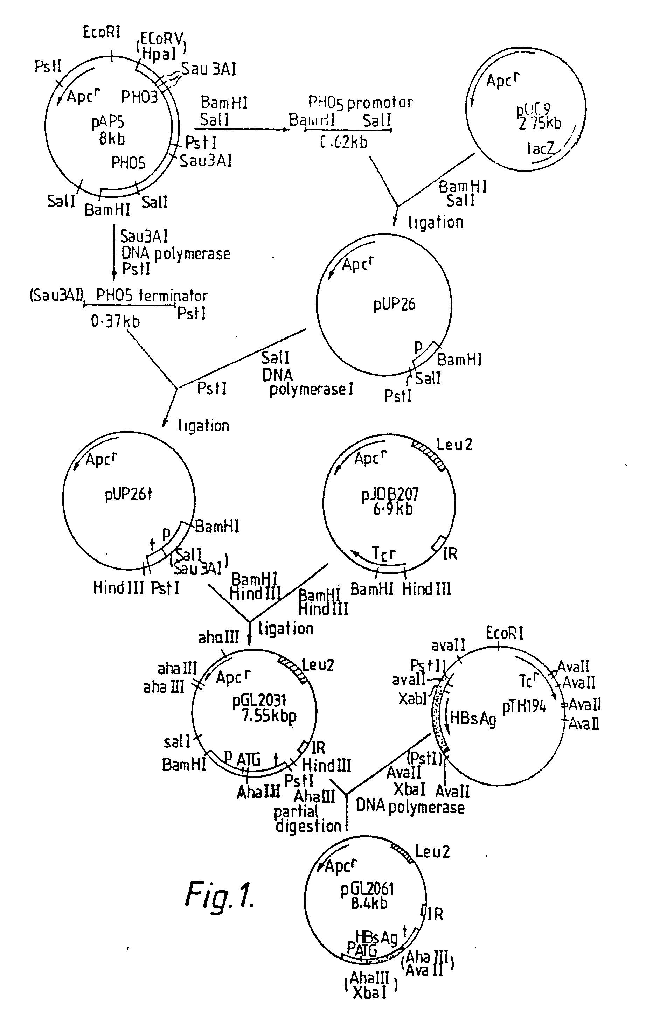

pGL2061 was constructed as described below (Fig. 1).

The yeast chromosome was extracted from *Saccharomyces cerevisiae* by the method of Cryer et al. (D. R. Cryer et al., Methods in Cell Biology (Academic Press), vol. X II, 39, 1975).

Then, the *PHO*5 gene was cloned on the basis of a report by Meyhack et al. (B. Meyhack et al., EMBO J., *1*, 675, 1982). Thus, the yeast chromosome obtained was digested with the restriction enzyme *BamH*I and, after 0.8% agarose gel electrophoresis, a DNA of about 5.1 kb in size was recovered. The DNA recovered was inserted into pUC9 at the *BamH*I site occurring in the polylinker sequence thereof and the insertion product was used for transformation of *E. coli* HB101. The transformants thus obtained were subjected to colony hybridization using a synthetic polynucleotide having the sequence GGCAAGGCATATACC as a probe. A plasmid (pUCPho) was prepared from a colony positive in said colony hybridization and the insert fragment was recovered therefrom through *BamH*I digestion and recloned at the *BamH*I site of pJDB207. Using the resultant plasmid, *Saccharomyces cerevisiae* AH22 was transformed, and the *PHO*5 activity (repressible acid phosphatase activity) was confirmed by the method of Toh-e et al. (A. Toh-e et al., J. Bacteriol., *113*, 727, 1973). The plasmid pUCPho having positive repressible acid phosphatase activity was digested with *BamH*I and *HPa*I and a 3.9 kb fragment was recovered by 1% agarose electrophoresis and inserted into pBR322 at the *BamH*I-*EcoR*V site thereof to give a plasmid, pAP5 (8 kb).

pAP5 was digested with *BamH*I and *Sal*I, and a *PHO*5 promoter fragment (0.62 kb) containing the ATG codon or *PHO*5 was isolated by 4% polyacrylamide gel electrophoresis (hereinafter referred to as PAGE) and inserted into a pUC9 at the *BamH*I-*Sal*I site occurring within the polylinker seqquence to give pUP26. Separately, pAP5 was digested with *Sau*3AI, followed by terminal repair using DNA polymerase I (Boehringer Mannheim) and further digestion with *Pst*I. The subsequent 4% PAGE (polyacrylamide gel

electrophoresis) gave a *PHO*5 terminator fragment (0.37 kb). pUP26 was digested with *Sal*I, terminally repaired with DNA polymerase I and further digested with *Pst*I. The digest was ligated with the above *PHO*5 terminator fragment to give pUP26t. pUP26t was digested with *Bam*HI and *Hind*III, and a 0.99 kb fragment containing the *PHO*5 promoter and terminator was recovered by 4% PAGE. This 0.99 kb fragment was inserted into pJDB207 at the *Bam*HI-*Hind*III site to give pGL2031.

The HBsAg gene-containing plasmid pTH194 obtained from Biogen (Geneva Switzerland) (Mackey et al., Proc. Natl. Acad. Sci. USA, *78*, 4510, 1981) was digested with *Xba*I and *Ava*II and an HBsAg gene fragment (0.86 kb) was recovered by 1.5% agarose gel electrophoresis. pGL2031 was partially digested with *Aha*III and Linear pGL2031 (7.55 kb) was recovered by 0.8% agarose gel electrophoresis. Into this pGL2031 fragment, there was inserted the previously prepared HBsAg gene *Xba*I-*Ava*II fragment after terminal repair thereof. Thus was constructed pGL2061.

## Example 1

The HBsAg-producing plasmid pGL2061 was partially digested with the restriction enzyme *Bst*EII (produced by NEB) (A total volume of 120 μl of a mixture containing 20 μg DNA, 60 units enzyme, 15 mM NaCl, 6 mM Tris-HCl (pH 7.9), 6 mM MgCl$_2$ and 6 mM 2-mercaptoethanol was incubated at 60°C and sampled in 20-μl portions after 0, 5, 15, 25 and 60 minutes of incubation and the reaction was terminated by adding SDS to a final concentration of 1%).

The partial digestion mixture portions were then subjected to 0.6% agarose gel electrophoresis to give an 8.4 kb pGL2061 fragment resultant from *Bst*EII cleavage at one-site.

Then, this pGL2061 fragment was completely digested with 16 units of the restriction enzyme *Sal*I (produced by Takara Shuzo) in a total volume of 25 μl of a mixture containing 175 mM NaCl, 10 mM Tris-HCl (pH 7.5), 7 mM MgCl$_2$, and 7 mM 2-mercaptoethanol at 37°C for 4 hours. The digest was electrophoresed on a 1% low-melting point agarose gel (produced by BRL) to give a DNA fragment having a size of 8 kb. Thus was obtained a miniaturized (modified) *PHO*5 promoter.

Both ends of this DNA fragment were repaired using 7.5 units of DNA polymerase I (produced by Boehringer Mannheim) in a total volume of a reaction medium containing 400 μM d-NTP, 20 mM NaCl, 7 mM Tris-HCl (pH 7.5) and 7 mM MgCl$_2$ at 20°C for 30 minutes.

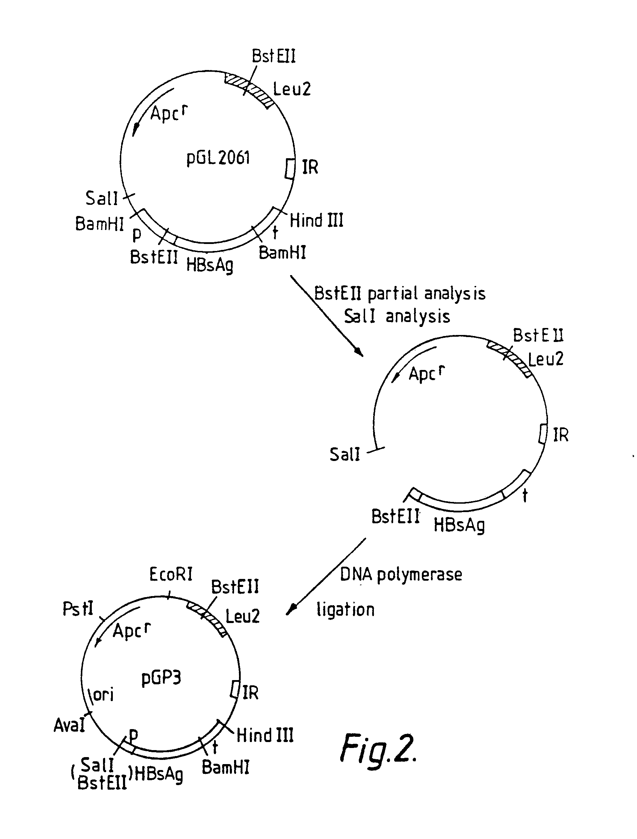

The repaired DNA fragment was ligated at 16°C over night in a total volume of 50 μl of a reaction mixture containing 175 units of T4 DNA ligase (produced by Takara Shuzo), 66 mM Tris-HCl (pH 7.5), 6.6 mM MgCl$_2$, 10 mM DTT and 1 mM ATP to give a plasmid pGP3 containing the pBR322-derived *Ava*I-*Sal*I fragment as a UAS (upstream activation sequence), the miniaturized (modified) *PHO*5 promoter and the structural gene for HBsAg (Fig. 2).

## Example 2

The plasmid pGP3 of Example 1 was used to transform *Saccharomyces cerevisiae* GRF18pho80 (α, *his, leu, trp, met, pho80)* and *S. cerevisiae* AH22 (a, *his, leu, can*) by the conventional method. The transformants obtained were cloned using leucine-free minimum medium plates (0.7% yeast nitrogen base (Difco), 2% dextrose, 1.5% agar). The cloned pGP3/GRF18pho80 and pGP3/AH22 were each shake-cultured on the above-mentioned minimum medium at 30°C for 2 days for use as a preculture. An 80-ml portion of the above-mentioned minimum medium was inoculated with 1% of each preculture, followed by shake culture at 30°C for 2 days. Cells were harvested by centrifugation, washed with one portion of physiological saline and suspended in a buffer (50 mM Tris-HCl (pH 7.5), 1 mM EDTA). This suspension was treated on a Tomy-Seiko model UR-200p sonicator at level 10 under ice cooling for 9 minutes and, then, centrifuged at 0°C and 13,000 × g for 10 minutes. The supernatant obtained was assayed for HBsAg by the RPHA method using ANTIHEBSEL (produced by The Geeen Cross Corp). The results thus obtained are shown in Table 2.

### TABLE 2

| Plasmid | Host | HBsAg activity ($2^n$) |
|---------|------|------------------------|
| pGL2061 | *S. cerevisiae* GRF18pho80 | 10 |
| pGP3 | Same as above | 8 |
| pGP3 | *S. cerevisiae* AH22 | 8 |

The thus-obtained miniaturized yeast promoter has enabled efficient expression of physiologically active substances in yeasts, providing a miniaturized *PHO*5 promoter so far unknown and facilitating recombinant DNA procedures. The promoter according to the invention has also enabled use of the *PHO*5 promoter in phosphate-added media and thus enabled production of physiologically active substances in more convenient medium systems.

**Claims**

1. A DNA sequence consisting essentially of that region of the yeast repressible acid phosphatase (PHO5) promoter which covers the 173 base pairs just upstream from the initiation codon for the PHO5 protein (without counting the initiation codon).

2. The DNA sequence of Claim 1, characterised by the following base sequence:

```
    -173                              -150
      .   .           .            .              .
        GTCACCTTACTTGGCAAGGCATATACCCATTTGG

                                              -100
              .              .            .           .
        GATAAGGGTAAACATCTTTGAATTGTCGAAATGAAACGTA

              .              .            .           .
        TATAAGCGCTGATGTTTTGCTAAGTCGAGGTTAGTATGGC

        -50
              .            .             .          .
        TTCATCTCTCATGAGAATAAGAACAACAACAAATAGAGCA

                          -1
                  .              .
        AGCAAATTCGAGATTACCA
```

3. A method of producing heterologous proteins which comprises utilizing, as the promoter, a DNA sequence consisting essentially of that region of the yeast repressible acid phosphatase (PHO5) promoter which covers the 173 base pairs just upstream from the initiation codon for the PHO5 protein.

4. A method of producing heterologous protein which comprises:
a) inserting into a plasmid, as promoter, a DNA sequence consisting essentially of the region of the yeast repressible acid phosphatase (PHO5) promoter which covers the 173 base pairs just upstream from the initiation codon for the PHO5 protein (excluding the initiation codon itself),
b) inserting a gene coding for a useful, physiologically-active substance into said plasmid at a site downstream from the promoter to construct a recombinant plasmid,
c) introducing the recombinant plasmid into a yeast strain to transform the yeast strain, and
d) cultivating the transformant to cause expression of the gene in the yeast strain.

5. The method of claim 4, wherein a heterologous protein is produced in a yeast by utilizing a PHO5 promoter region having the following base sequence

```
    -173                              -150
      .   .           .            .              .
        GTCACCTTACTTGGCAAGGCATATACCCATTTGG

                                              -100
              .              .            .           .
        GATAAGGGTAAACATCTTTGAATTGTCGAAATGAAACGTA

              .              .            .           .
        TATAAGCGCTGATGTTTTGCTAAGTCGAGGTTAGTATGGC

        -50
              .            .             .          .
        TTCATCTCTCATGAGAATAAGAACAACAACAAATAGAGCA

                          -1
                  .              .
        AGCAAATTCGAGATTACCA
```

6. The method of Claim 3 or Claim 4, wherein a pBR322 derived *Aval-Sal*I fragment is used as an upstream activation site (UAS) of the *PHO*5 promoter.

7. A method of producing HBsAg which comprises partially digesting the HBsAg-producing plasmid pGL2061 with the restriction enzyme *Bst*EII to give a pGL2061 fragment about 8.4 kb in size as resulting from one-site cleavage caused by *Bst*EII, subjecting said fragment to complete digestion with the restriction enzyme *Sal*I to give a DNA fragment having a size of about 8 kb and bearing a miniaturized *PHO*5 promoter and the HBsAg gene, repairing both ends of this DNA fragment using DNA polymerase I, subjecting the DNA fragment to ligation using T4 DNA ligase to thereby construct a plasmid (pGP3), introducing this plasmid into a strain of yeast for transformation thereof and cultivating the resultant transformant to thereby cause expression of the HBsAg gene. .

## Patentansprüche

1. DNA-Sequenz, bestehend im wesentlichen aus dem Bereich des Hefe-Promotors der repressiblen Säure-Phosphatase (*PHO*5), der die 173 Basen-Paare unmittelbar oberhalb des Initiierungs-Codons für das *PHO*5-Protein (ohne Zählung des Initiierungs-Codons) umfaßt.

2. DNA-Sequenz nach Anspruch 1, gekennzeichnet durch die folgende Basen-Sequenz:

```
         -173                        -150
          •   •         •          •        •        •
          GTCACCTTACTTGGCAAGGCATATACCCATTTGG

                                             -100
                  •           •          •        •
          GATAAGGGTAAACATCTTTGAATTGTCGAAATGAAACGTA

                  •           •          •        •
          TATAAGCGCTGATGTTTTGCTAAGTCGAGGTTAGTATGGC

          -50
                  •           •          •        •
          TTCATCTCTCATGAGAATAAGAACAACAACAAATAGAGCA

                       -1
                  •             •
          AGCAAATTCGAGATTACCA
```

3. Verfahren zur Herstellung von heterologen Proteinen, umfassend die Verwendung einer DNA-Sequenz als Promotor, die im wesentlichen aus dem Bereich des Hefe-Promotors der repressiblen Säure-Phosphatase (*PHO*5), der die 173 Basen-Paare unmittelbar oberhalb des Initiierungs-Codons für das *PHO*5-Protein umfaßt, besteht.

4. Verfahren zur Herstellung eines heterologen Proteins, umfassend

a) das Einfügen einer DNA-Sequenz als Promotor in ein Plasmid, die im wesentlichen aus dem Bereich des Hefe-Promotors de repressiblen Säure-Phosphatase (*PHO*5), der die 173 Basen-Paare unmittelbar oberhalb des Initiierungs-Codons für das *PHO*5-Protein umfäßt, besteht (ohne Zählung des Initiierungs-Codons),

b) das Einfügen eines für eine brauchbare, physiologisch aktive Substanz codierenden Gens in das Plasmid an einer Stelle unterhalb des Promotors, um ein rekombinantes Plasmid zu konstruieren,

c) das Einführen des rekombinanten Plasmids in einen Hefe-Stamm, um den Hefe-Stamm zu transformieren, und

d) das Kultivieren der Transformante, um die Expression des Gens in dem Hefe-Stamm zu bewirken.

5. Verfahren nach Anspruch 4, worin ein heterologes Protein in einer Hefe dadurch erzeugt wird, daß ein *PHO*5-Promotor-Bereich benutzt wird, der die folgende Basen-Sequenz besitzt:

```
        -173                         -150
         .   .           .        .       .
             GTCACCTTACTTGGCAAGGCATATACCCATTTGG

                                             -100
                  .           .        .       .
             GATAAGGGTAAACATCTTTGAATTGTCGAAATGAAACGTA

                  .           .        .       .
             TATAAGCGCTGATGTTTTGCTAAGTCGAGGTTAGTATGGC

        -50
                  .           .        .       .
             TTCATCTCTCATGAGAATAAGAACAACAACAAATAGAGCA

                           -1
                  .                   .
             AGCAAATTCGAGATTACCA
```

6. Verfahren nach Anspruch 3 oder Anspruch 4, worin ein von pBR322 abgeleitetes *Aval-Sal*l-Fragment als oberhalb gelegenes Aktivierungszentrum (UAS) des *PHO*5-Promotors verwendet wird.

7. Verfahren zur Erzeugung von HBsAg, umfassend das partielle Verdauen des HBsAg-produzierenden Plasmids pGL2061 mit dem Restriktionsenzym *Bst*EII zur Bildung eines pGL2061-Fragments mit einer Größe von etwa 8,4 kb, wie es aus der durch *Bst*EII verursachten Einzentren-Spaltung resultiert, die Einwirkung des Restriktionsenzyms *Sal*l auf das Fragment zur vollständigen Verdauung desselben zur Bildung eines DNA-Fragments, das eine Größe von etwa 8 kb hat und einen miniaturisierten *PHO*5-Promotor und das HBsAg-Gen trägt, das Reparieren der beiden Enden dieses DNA-Fragments unter Verwendung von DNA-Polymerase I, das Einwirkenlassen der Ligation auf das DNA-Fragment unter Verwendung von T4-DNA-Ligase, wodurch ein Plasmid (pGP3) konstruiert wird, das Einführen des Plasmids in einen Hefe-Stamm zur Transformation desselben und das Kultivieren der resultierenden Transformante, um die Expression des HBsAg-Gens zu verursachen.

**Revendications**

1. Séquence d'ADN consistant essentiellement en la région du promoteur de phosphatase acide répressible (*PHO*5) de levure qui couvre les 173 paires de bases juste en amont du codon d'initiation pour la protéine *PHO*5 (sans compter le codon d'initiation).

2. La séquence d'ADN de la revendication 1, caractérisée par la séquence de bases suivante:

```
        -173                         -150
         .   .           .        .       .
             GTCACCTTACTTGGCAAGGCATATACCCATTTGG

                                             -100
                  .           .        .       .
             GATAAGGGTAAACATCTTTGAATTGTCGAAATGAAACGTA

                  .           .        .       .
             TATAAGCGCTGATGTTTTGCTAAGTCGAGGTTAGTATGGC

        -50
                  .           .        .       .
             TTCATCTCTCATGAGAATAAGAACAACAACAAATAGAGCA

                           -1
                  .                   .
             AGCAAATTCGAGATTACCA
```

7

3. Méthode de production de protéines hétérologues, qui comprend l'utilisation, comme promoteur, d'une séquence d'ADN consistant essentiellement en la région du promoteur de phosphatase acide répressible (PHO5) de levure qui couvre les 173 paires de bases juste en amont du codon d'initiation pour la protéine PHO5.

4. Méthode de production de protéines hétérologues, qui comprend:

a) insertion dans un plasmide, comme promoteur, d'une séquence d'ADN consistant essentiellement en la région du promoteur de phosphatase acide répressible (PHO5) de levure, qui couvre les 173 paires de bases juste en amont du codon d'initiation pour la protéine PHO5 (excluant le codon d'initiation lui-même),

b) insertion d'un gène codant pour une substance physiologiquement active utile dans ledit plasmide en un site en aval du promoteur pour construire un plasmide recombinant,

c) introduction du plasmide recombinant dans une souche de levure pour transformer la souche de levure, et

d) culture du transformant pour provoquer l'expression du gène dans la souche de levure.

5. La méthode de la revendication 4, dans laquelle une protéine hétérologue est produite dans une levure en utilisant une région de promoteur de PHO5 ayant la séquence de bases suivante:

$$-173 \qquad\qquad -150$$

$$\text{GTCACCTTACTTGGCAAGGCATATACCCATTTGG}$$

$$-100$$

$$\text{GATAAGGGTAAACATCTTTGAATTGTCGAAATGAAACGTA}$$

$$\text{TATAAGCGCTGATGTTTTGCTAAGTCGAGGTTAGTATGGC}$$

$$-50$$

$$\text{TTCATCTCTCATGAGAATAAGAACAACAACAAATAGAGCA}$$

$$-1$$

$$\text{AGCAAATTCGAGATTACCA}$$

6. La méthode de la Revendication 3 ou de la Revendication 4, dans laquelle un fragment AvaI-SalI dérivé de pBR322 est utilisé comme site d'activation en amont (UAS) du promoteur de PHO5.

7. Méthode de production de HBsAg qui comprend la digestion partielle du plasmide pGL2061 produisant HBsAg par l'enzyme de restriction BstII pour donner un fragment de pGL2061 d'environ 8,4 kb comme il ressort du clivage en un site provoqué par BstII, la digestion complète dudit fragment par l'enzyme de restriction SalI pour donner un fragment d'ADN ayant une taille d'environ 8 kb et portant un promoteur de PHO5 miniaturisé et le gène de HBsAg, la réparation des deux extrémités de ce fragment d'ADN en utilisant l'ADN polymérase I, la ligation dudit fragment d'ADN en utilisant la T4-ADN ligase pour construire ainsi un plasmide (pGP3), en l'introduction de ce plasmide dans une souche de levure pour la transformation de celle-ci et la mise en culture du transformant obtenu pour provoquer ainsi l'expression du gène de HBsAg.

EP 0 216 573 B1

Fig.1.

*Fig.2.*